# EUROPEAN PATENT APPLICATION

(11) **EP 4 541 806 A1**
(43) Date of publication of application: **23.04.2025**
(21) Application number: 23824257.2
(22) Date of filing: 15.06.2023
(51) Int. Cl.: C07K 14/005, A61K 39/215, A61P 31/14, G01N 33/569

(54) **MODIFIED CORONAVIRUS SPIKE ANTIGEN PROTEIN AND USES THEREOF**

(30) Priority: 16.06.2022 KR 20220073355
(71) Applicant: UIF (University Industry Foundation), Yonsei University, Seoul 03722 (KR)
(72) Inventor: OH, Jong-Won, Goyang-si, Gyeonggi-do 10325 (KR); SEO, Han Young, Seoul 03643 (KR)
(74) Representative: Ostertag & Partner Patentanwälte mbB
(86) International application number: PCT/KR2023/008308
(87) International publication number: WO 2023/244044

(57) **Abstract**

One aspect relates to a modified coronavirus spike antigen protein and uses thereof. It was confirmed that a spike antigen protein of coronavirus, according to one aspect, exhibited suppression of cell membrane fusion ability and improvement in safety by modifying two protein cleavage sites present in a coronavirus spike protein of the coronavirus. In addition, inoculation with a vaccine using said antigen protein induces the production of a large amount of neutralizing antibodies to inhibit the invasion of the coronavirus into cells, thereby suppressing viral proliferation. Accordingly, the present invention can be used in various industries and markets, such as prevention of coronavirus infection, alleviation and treatment of symptoms, infection diagnosis, etc.

## Description

### [Technical Field]

The present invention relates to a modified coronavirus spike antigen protein and a use thereof.

### [Background Art]

A new human coronavirus that was first found in December 2019 in Wuhan province, China, is the causative agent of coronavirus disease 19 (COVID-19) respiratory disease, which was named SARS-CoV-2 in February 2020 due to its similarities to SARS-CoV that was found in 2002.

SARS-CoV-2 is an enveloped virus with a single-stranded, positive-sense RNA genome that produces one long polyprotein encoded by ORF1a/b, nine structural proteins, and five accessory proteins. The polyprotein is processed to produce 16 non-structural proteins by viral and host proteases after translation. The five accessory proteins function to suppress the host's immune response or to help the virus replicate and assemble, and the four structural proteins that constitute the viral structure include spike (S), envelope, membrane, and nucleocapsid proteins.

The spike protein is a glycoprotein that consists of more than 1,200 amino acids and is an important factor for viral entry into host cells. The S protein consists of S1 and S2 domains. Its cleavage into the S1 and S2 domains is carried out by the furin protease present in the Golgi, and uncleaved full-length S and its cleaved products, S1 and S2 domains, can be loaded on the surface of viral particles or transferred into the cell membrane without being separated. The S1 domain of the uncleaved S protein or the S1 domain still attached to S2 after cleavage plays an essential role in the entry of SARS-CoV into cells via an ACE2 receptor. After binding to the receptor, the S2 domain can be cleaved near the S2' position (the residue of arginine 815) by a host protease, such as TMPRSS2 or cathepsin, at the cell membrane or within endosomes, and a fusion peptide exposed during this process induces membrane fusion.

Several types of vaccines have been developed against SARS-CoV-2 and are either in clinical trials or have been authorized for emergency use. As of March 2023, a total of 81% of the SARS-CoV-2 vaccines, including 17 spike protein-encoded DNA vaccines (9%), 43 mRNA vaccines (24%), and 29 viral vector vaccines (16%), which account for 49% of the total vaccines, and 59 spike-derived protein vaccines (32%), use spike proteins as an antigen.

A representative design of the spike protein antigen for the coronavirus vaccine currently used is the "2P design," in which both the lysine residue at amino acid position 986 and the valine residue at amino acid position 987 in the S2 domain are substituted with proline to keep a spike protein in a prefusion conformational state. In the 2P design, the furin cleavage site of the spike protein is retained, which can lead to the separation of the S1 subunit from the S2 domain of the spike protein. The detached S1 domain has the potential to induce an inflammatory response, raising safety concerns for the vaccine. In addition, the immunogenicity of a prefusion conformational modified spike protein with an altered structure may differ from that of a wild-type spike protein. Accordingly, designing a spike antigen with minimal modifications that retain its receptor recognition function while maintaining immunogenicity is crucial for developing a vaccine that is both safe and capable of providing enhanced immune protection.

Therefore, to address these challenges, the present inventors have developed a modified coronavirus spike antigen protein in which protein cleavage sites in the spike protein are altered to prevent cleavage by intracellular, membrane-associated, or extracellular proteases. This coronavirus spike antigen modification technology, as described in the patent, enhances safety and leads to the generation of high-titer spike-binding antibodies and potent neutralizing antibodies.

### [Disclosure]

### [Technical Problem]

The present invention is related to providing a spike antigen protein of coronavirus in which a host protease cleavage site of the spike protein of coronavirus is modified.

The present invention is also related to providing a recombinant vector for expressing an antigen protein of coronavirus, which includes a gene encoding the spike antigen protein of coronavirus.

The present invention is also related to providing a transformant, which is transformed with the recombinant vector.

The present invention is also related to providing a vaccine composition against a coronavirus, which includes the spike antigen protein of coronavirus or a gene encoding the same.

The present invention is also related to providing a pharmaceutical composition for preventing or treating a coronavirus infection, which includes the spike antigen protein of coronavirus.

The present invention is also related to providing a composition for diagnosing a coronavirus infection, which includes the spike antigen protein of coronavirus.

The present invention is also related to providing a coronavirus infection diagnostic kit, which includes the composition.

The present invention is also related to providing a method of introducing an antigen gene of coronavirus, which includes transforming a subject with a gene encoding the spike antigen protein of coronavirus.

The present invention is also related to providing a method of preventing or treating coronavirus infection, which includes administering the spike antigen protein of coronavirus to an individual in need thereof.

The present invention is also related to providing the use of the spike antigen protein of coronavirus to prepare a drug for preventing or treating a coronavirus infection.

### [Technical Solution]

One aspect of the present invention provides a spike antigen protein of coronavirus in which a host protease cleavage site of the spike protein of coronavirus is modified.

The term "coronavirus" refers to a single-stranded, positive-sense RNA virus, which belongs to the family of *Coronaviridae,* and has a spherical envelope and a size of approximately 100 to 220 nm.

The term "spike protein" refers to a protruding structure arranged on the viral surface, 10 to 15 nm in length, that binds to a protein receptor on the cell membrane, enabling the virus to enter the host cell.

The term "host protease" refers to the protease of a host infected by a coronavirus.

In one embodiment, the cleavage site may be at least one selected from the cleavage sites of the furin protease in the sequences of the S1 and S2 domains of the spike protein of coronavirus and the cleavage site of the protease in the S2' sequence thereof.

The term "furin protease" refers to an enzyme that hydrolyzes peptide bonds between the amino acids constituting the protein.

In one embodiment, the protease acting on the cleavage site thereof in the S2' sequence may be any host protease without limitation.

In one embodiment, the coronavirus may be at least one selected from the group consisting of severe acute respiratory syndrome coronavirus (SARS-CoV), severe acute respiratory syndrome coronavirus 2 (SARS-CoV-2), Middle East respiratory syndrome coronavirus (MERS-CoV), human coronavirus 229E (HCoV-229E), and variants thereof.

In one embodiment, the coronavirus may be at least one selected from the group consisting of SARS-CoV-2 and variants thereof.

The term "severe acute respiratory syndrome coronavirus (SARS-CoV)" refers to a viral strain causing SARS, and an enveloped, positive-sense, single-stranded RNA virus, infecting the lung epithelial cells of humans, bats, and Asian civet cats.

The term "severe acute respiratory syndrome coronavirus 2 (SARS-CoV-2)" refers to a type 2 SARS coronavirus, and an agent with a positive-sense, single-stranded RNA gene that causes coronavirus infection-19, which is a zoonotic and human-to-human infectious disease.

The term "Middle East respiratory syndrome coronavirus (MERS-CoV)" refers to a species of zoonotic coronavirus that infects humans and camels, and an agent that causes severe respiratory diseases such as SARS-CoV.

The term "human coronavirus (HCoV-229E)" refers to a species of coronavirus that infects humans and bats, and an enveloped, positive-sense, single-stranded RNA virus that causes the common cold.

The term "variant" refers to a virus whose genome has been modified compared to the representative species, and the coronavirus variant may have 90%, 91%, 92%, 93%, 94%, 95%, 96%, 97%, 98%, or 99% genomic or amino acid homology with the above coronavirus.

For example, variants of the SARS-CoV-2 may be variants of concern, and may be specifically at least one selected from the group consisting of alpha, beta, gamma, delta, and omicron variants, and sub-variants thereof.

In one embodiment, the coronavirus may be SARS-CoV-2, with the furin protease cleavage site located at amino acid 685 of the spike protein and the protease cleavage site in the S2' sequence located at amino acid 815 of the spike protein. In addition, the positions of these amino acids may be based on the Wuhan-Hu-1 reference sequence.

In one embodiment, the amino acid 685 in the cleavage site of the furin protease may be substituted with at least one amino acid selected from the group consisting of glycine, alanine, valine, leucine, isoleucine, threonine, serine, cysteine, methionine, aspartic acid, asparagine, glutamic acid, glutamine, lysine, histidine, phenylalanine, tyrosine, tryptophan, and proline.

In one embodiment, amino acid 685 of the cleavage site of the furin protease may be substituted with at least one amino acid selected from the group consisting of serine, histidine, proline, and valine.

Among these, it may be a modified spike protein antigen substituted with serine, which, despite the modification, has a higher ACE2 receptor recognition ability than the wild-type.

In one embodiment, in the cleavage site of the protease in the S2' sequence, the amino acid 815 of the spike protein may be substituted with at least one selected from the group consisting of glycine, alanine, valine, leucine, isoleucine, threonine, serine, cysteine, methionine, aspartic acid, asparagine, glutamic acid, glutamine, phenylalanine, tyrosine, tryptophan, and proline.

In one embodiment, in the cleavage site of the protease in the S2' sequence, the amino acid 815 of the spike protein may be substituted with at least one selected from the group consisting of valine, methionine, proline, and alanine.

In one embodiment, the spike antigen protein of the modified coronavirus may exhibit increased affinity to angiotensin-converting enzyme 2 (ACE2).

The term "angiotensin-converting enzyme 2 (ACE2)" refers to a metallic protease that encodes 805 amino acids and plays a role as a receptor that acts as a cell entry point of coronavirus.

In one embodiment, the spike antigen protein of the modified coronavirus may increase receptor-binding domains in a spike trimer.

In one embodiment, the spike antigen protein of the modified coronavirus may form a vaccine antigen structure.

The spike antigen protein of the modified coronavirus may produce spike-binding antibodies and neutralizing antibodies, which can protect against coronavirus infection and induce T cell immune activity because of the structure of the vaccine antigen.

In one embodiment, the spike antigen protein of the modified coronavirus may inhibit the production of the free form of the S1 domain.

The free S1 domain may stimulate toll-like receptor 4 (TLR4) to induce an inflammatory response.

The term "toll-like receptor 4 (TLR4)" refers to an innate immune response-inducing receptor.

In one embodiment, the spike antigen protein of the modified coronavirus may inhibit cell membrane fusion.

The term "cell membrane fusion" refers to a very important essential action for viruses to enter host cells through a lipid bilayer, and means the merging of the viral and host cell membranes into a single unified structure.

In one embodiment, the spike antigen protein of the modified coronavirus may inhibit the intracellular entry of a virus or the intracellular delivery of a viral gene.

The term "intracellular entry" refers to the entry of a virus into cells.

The term "intracellular delivery" refers to the process in which viral genes are delivered into the cytoplasm through the cell membrane or endosomal lipid bilayer by membrane fusion when a modified spike protein is present on the surface of a viral particle or natural and artificial membrane structure.

In one embodiment, as a result of confirming the ability to induce cell membrane fusion by substituting the amino acid 685 at the S1/S2 cleavage site and the amino acid 815 at the S2' cleavage site of the spike protein of SARS-CoV-2 or a variant thereof with serine and alanine, respectively, it was confirmed that cell fusion is significantly inhibited, preventing the production of large multinucleated cells (refer to Example 8).

Another aspect of the present invention provides a recombinant vector for expressing an antigen protein of coronavirus, which includes a gene encoding the spike antigen protein of coronavirus.

The "coronavirus," and "spike antigen protein" may be in the above-described range.

The term "vector" refers to any vehicle for gene cloning and/or transfer into a host cell. The vector may be a replicon to which different DNA fragments may be inserted, resulting in the replication of the inserts. In addition, the vectors may include viral and non-viral vehicles for introducing genes into host cells *in vitro, ex vivo,* or *in vivo.*

The term "replicon" refers to any genetic unit (e.g., a plasmid, a phage, a cosmid, a chromosome, or a virus) that functions as a self-replicating unit of DNA *in vivo,* meaning that it can replicate independently through its own regulatory mechanisms.

In one embodiment, the vector is not particularly limited as long as it can replicate in a host cell, and any vector known in the art can be used. Examples of commonly used vectors include plasmids, cosmids, viruses, and bacteriophages, which are in either a natural or recombinant state. For example, pWE15, M13, MBL3, MBL4, IXII, ASHII, APII, t10, t11, Charon4A, and Charon21A may be used as phage vectors or cosmid vectors, and pBR type, pUC type, pBluescriptII type, pGEM type, pTZ type, pCL type, and pET type may be used as plasmid vectors. Specifically, pDZ, pACYC177, pACYC184, pCL, pECCG117, pUC19, pBR322, pMW118, pCC1BAC, pSKH, pRS-413, pRS-414, pRS-415, pDSK519, pDSK, and pDART vectors may be used, but the present invention is not limited thereto.

In addition, there are no specific restrictions on the choice of vector; any known expression vector can be used, and a polynucleotide encoding a target protein can be introduced into a chromosome using a vector designed for chromosomal integration in cells. The insertion of the polynucleotide into the chromosome may be accomplished by any method known in the art, for example, homologous recombination, but the method is not limited thereto. A selection marker for checking whether the chromosome has been inserted may be further included. A selection marker is used to select cells transformed with a vector, i.e., to check whether a target nucleic acid molecule has been inserted, and markers that confer selectable phenotypes such as drug resistance, nutritional requirements, resistance to cytotoxic agents, or the expression of surface proteins may be used. In an environment treated with a selective agent, only cells expressing the selection marker survive or exhibit other phenotypic traits, so transformed cells may be selected.

Still, another aspect of the present invention provides a transformant that is transformed with the recombinant vector.

The "recombinant vector" may be within the above-described range.

The term "transformation" means a change in the genetic properties of an organism due to DNA given from the outside-in other words, a phenomenon in which DNA, a type of nucleic acid extracted from the cells of a certain lineage of an organism, is injected into the living cells of another lineage of an organism, and thus changes its genotype.

The transformation method includes any method of introducing nucleic acid into a cell and may be performed by selecting an appropriate standard technique known in the art depending on host cells. The technique may be, for example, electroporation, CaPO₄ precipitation, CaCl₂ precipitation, microinjection, a polyethyleneglycol (PEG) method, an EAE-dextran method, a cationic liposome method, or a lithium acetate-DMSO method, but the present invention is not limited thereto.

The term "transformant" refers to a transgenic plant or animal produced by transformation, and the transformant may include a genetic recombinant produced by inducing a modification or mutation of a specific gene using a genetic recombination technology.

In one embodiment, the transformant may be appropriately selected and used by those skilled in the art without limitation as long as it is a cell type that can be used for transformation known in the field.

Yet another aspect of the present invention provides a vaccine composition against a coronavirus, which includes the spike antigen protein of coronavirus or a gene encoding the same.

The "coronavirus," "spike antigen protein," or "coding gene" may be within the above-described range.

In one embodiment, the vaccine composition may enhance the production of neutralizing antibodies.

The term "neutralizing antibody" refers to an antibody that binds to a pathogen or infectious particle, inhibiting its entry into host cells through a receptor and preventing infection. The neutralizing antibody is part of the adaptive immune response to viruses, intracellular bacteria, and microbial toxins, and it is produced in response to the specific surface structures of infectious particles, preventing its interaction with host cells and providing protective immunity. Generally, when a vaccine is administered, binding antibodies and neutralizing antibodies against target antigens are produced in the body. Binding antibodies have the function of recognizing infectious cells and labeling infectious agents, facilitating their removal by immune cells. In addition, binding antibodies can activate the complement system to lyse infected cells and damage virus particles. On the other hand, neutralizing antibodies specifically bind to target virulent antigens, reducing pathogenicity, or bind to viral particles, triggering antigen-specific immune responses that block viral entry into host cells.

The term "vaccine composition" refers to a biological agent that contains an antigenic material that induces immunity in an organism, and also refers to an immunogen that is administered or injected to prevent infection and establish protective immunity. The vaccine composition can elicit both cellular immune responses, e.g., cytotoxic T lymphocyte (CTL), and humoral immune responses, e.g., enhanced systemic or local antibody production. The subject receiving the vaccine can be any living organism susceptible to a coronavirus, including humans, rats, mice, and livestock. Specifically, the subject is often a mammal such as a human.

In one embodiment, the vaccine composition may further include a pharmaceutically acceptable carrier. The term "pharmaceutically effective amount" refers to a sufficient amount to achieve preventive, alleviating, or therapeutic efficacy against a disease or pathological symptom caused by a virus. Pharmaceutically acceptable carriers that may be included in the vaccine composition are commonly used in formulations, and include lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, talc, magnesium stearate, and mineral oil, but the present invention is not limited thereto.

In addition, the vaccine composition may be used in an oral formulation such as a powder, a granule, a tablet, a capsule, a suspension, an emulsion, a syrup, or an aerosol, or a sterilized injectable solution according to a conventional method. The vaccine composition may be prepared using a diluent or an excipient such as a filler, a thickening agent, a binder, a wetting agent, a disintegrant, and a surfactant, which are commonly used. Examples of a solid preparation for oral administration include a tablet, a pill, a powder, a granule, and a capsule, and such a solid preparation may be formulated by mixing at least one excipient, for example, starch, calcium carbonate, sucrose, lactose, or gelatin, with the lecithin-like emulsifier. In addition, other than simple excipients, a lubricant such as magnesium stearate or talc may be used. A liquid preparation for oral administration may be a suspension, a liquid for internal use, an emulsion or a syrup, and a generally used simple diluent such as water or liquid paraffin, as well as various types of excipients, for example, a wetting agent, a sweetener, a flavor, and a preservative, may be included. Preparations for non-oral administration include a sterile aqueous solution, a non-aqueous solvent, a suspending agent, an emulsifier, and a freeze-dried preparation. As a non-solubilizer and a suspending agent, propylene glycol, polyethylene glycol, a vegetable oil such as olive oil, or an injectable ester such as ethyl oleate may be used.

The dosage form of the vaccine composition may be in the form of enteric-coated units, preparations for intraperitoneal, intramuscular, or subcutaneous administration, aerosol spray, or formulations for oral or intranasal use. The vaccine composition may be administered in any conventional manner via oral, rectal, topical, intravenous, intraperitoneal, intramuscular, intra-arterial, transdermal, intranasal, inhalation, intraocular, or intradermal routes.

The vaccine composition may be parenterally administered. Parenteral administration means administration methods, including intravenous, intramuscular, intraperitoneal, intrasternal, percutaneous, and intra-arterial injections and infusions. For parenteral administration of a vaccine, the vaccine composition has to be formulated in a unit dosage form by being mixed with agents that are non-toxic to a recipient and able to be combined with components of other agents under a preferred purity within a pharmaceutically acceptable concentration and dosage.

In addition, the vaccine composition may be administered to a subject in need thereof at a pharmaceutically effective amount. An appropriate dosage of the vaccine composition may be determined in various ways by factors such as a formulation method, an administration mode, a subject's age, body weight, sex, and pathological condition, food, administration time, an administration route, an excretion rate, and sensitivity to a response.

In one embodiment, as a result of confirming immunogenicity in mice by lipid nanoparticle (LNP) formulation of mRNA of SARS-CoV-2 Delta variant (B.1.617.2) wild-type spike protein, and mRNA which expresses a modified spike antigen protein in which the amino acid residues at the S1/S2 cleavage site and the S2' cleavage site were substituted with serine and alanine, respectively, it was confirmed that the ND₅₀ value, which is the serum dilution fold indicating the 50% virus neutralization effect, was high, exceeding 1,000 (see Example 9).

In addition, as a result of confirming the immunogenicity of an mRNA vaccine that expresses a modified spike protein in which the amino acid residues at the S1/S2 cleavage site and the S2' cleavage site of each of spike proteins of the SARS-CoV-2 variants (omicron BA.1 and BA.5) were substituted with serine and alanine, respectively, it was confirmed that the reciprocal serum endpoint titer, which is the serum dilution fold indicating the level of antibodies which recognizes full-length spikes, can induce an immune response as high as 10⁶ to 10⁷ (see Example 9).

In another embodiment, as a result of evaluating the prevention efficacy against viral infection after inoculating a mouse with the mRNA vaccine expressing the SARS-CoV-2 omicron BA.5 modified spike antigen, it was confirmed that no viral infection was observed in the lungs of the vaccinated mice (BALB/c) (see Example 10).

In still another embodiment, as a result of confirming the immunogenicity of the mRNA vaccine expressing a modified spike protein in which the amino acid residues at the S1/S2 cleavage site and the S2' cleavage site of each spike protein of a SARS-CoV-2 variant (omicron sub-variant XBB.1.5) are substituted with serine and methionine, respectively, it was confirmed that all reciprocal serum endpoint titers, which are serum dilution folds indicating the levels of binding antibodies that recognize full-length spikes and receptor-binding domains (RBDs), can induce high immune responses exceeding 10⁷. In addition, as a result of analyzing neutralizing antibodies using a pseudotyped virus that expresses the XBB.1.5 spike protein on the surface of the viral particle, it was confirmed that the ND₅₀ value, which is the serum dilution fold that indicates the 50% virus neutralization effect, was high, exceeding 10,000 (see Example 12).

Yet another aspect of the present invention provides a pharmaceutical composition for preventing or treating coronavirus infection, which includes the spike antigen protein of coronavirus.

The "coronavirus," "spike antigen protein," or "coronavirus infection" may be within the above-described range.

The term "prevention" refers to all actions that inhibit infection with coronavirus or delay the onset of coronavirus infection in an individual by administering the pharmaceutical composition.

The term "treatment" refers to all actions involved in improving or beneficially changing symptoms of coronavirus infection in an individual by administering the pharmaceutical composition.

The term "administration" refers to the introduction of a predetermined material to an individual by a proper method.

In addition, the pharmaceutical composition may include an active ingredient alone, or may be provided together with at least one pharmaceutically acceptable carrier, excipient, or diluent.

The term "pharmaceutically acceptable" means that there is no toxicity to cells or a human exposed to the composition.

Specifically, the carrier may be, for example, a colloidal suspension, a powder, a saline, a lipid, liposomes, microspheres, or nanospheres. These carriers may form a complex with a vehicle or may be involved in forming the complex, and may be transported *in vivo* using carrier systems known in the art, including lipids, liposomes, microparticles, gold, nanoparticles, polymers, condensation agents, polysaccharides, polyamino acids, dendrimers, saponin, adsorption-enhancing materials, and fatty acids.

When the pharmaceutical composition is prepared, it may be formulated using diluents or excipients, such as a lubricant, a sweetener, a flavor, an emulsifier, a suspending agent, a preservative, a filler, a bulking agent, a binder, a wetting agent, a disintegrant, and a surfactant, which is conventionally used. Preparations for parenteral administration may include a sterile aqueous solution, a non-aqueous solvent, a suspending agent, an emulsifier, a freeze-dried preparation, or a suppository. As a non-aqueous solvent or a suspending agent, propylene glycol, polyethylene glycol, vegetable oil such as olive oil, or an injectable ester such as ethyl oleate may be used. As a suppository base, Witepsol, macrogol, Tween 61, cacao butter, laurin butter, or glycerogelatin may be used, and when manufactured in the form of eye drops, a known diluent or excipient may be used.

When mRNA is used for antigen expression, liposomes, LNPs, microneedles, or extracellular vesicles derived from animal/microbial/plant cells, which can maintain the stability of mRNA and be transferred to the cytoplasm, may be used as vehicles. Alternatively, mRNA may be transferred by a physical method such as electroporation.

The pharmaceutical composition may be administered at a pharmaceutically effective amount. The term "pharmaceutically effective amount" used herein refers to an amount sufficient for treating a disease at a reasonable benefit/risk ratio applicable for medical treatment, and an effective dosage may be determined based on parameters including the type and severity of a patient's disease, the drug activity, the patient's sensitivity to the drug, the administration time and route, the excretion rate, the duration of treatment, and the drugs simultaneously used, and other factors well known in the medical field.

In one embodiment, the pharmaceutical composition may be administered once or in several divided doses a day. For example, the pharmaceutical composition may be administered every other day or once a week. Specifically, the pharmaceutical composition may be administered at 0.001 to 1000 mg/kg/day, and more specifically, 0.1 to 100 mg/kg/day. The administration may be performed once or in several divided doses a day.

Yet another aspect of the present invention provides a composition for diagnosing coronavirus infection, which includes the spike antigen protein of coronavirus.

The "coronavirus" and "spike antigen protein" may be within the above-described ranges.

The term "coronavirus infection" refers to a severe respiratory syndrome caused by a coronavirus, and in one embodiment, the coronavirus may be severe acute respiratory syndrome coronavirus 1 (SARS-CoV1), severe acute respiratory syndrome coronavirus 2 (SARS-CoV-2), human coronavirus 229E (HCoV-229E), and variants thereof.

The term "variant" refers to a modified genome compared to a representative virus species, and in one embodiment, the coronavirus variant may have 90%, 91%, 92%, 93%, 94%, 95%, 96%, 97%, 98%, or 99% genomic or amino acid homology with the coronavirus.

For example, variants of the SARS-CoV-2 may be variants of concern, and specifically, may be Alpha, Beta, Gamma, Delta, and Omicron variants, and sub-variants thereof.

The term "diagnosis," in a broad sense, means judging a patient's condition in all aspects. The content of the judgment includes the name of the disease, the cause, the type, the severity, the detailed state of the condition, and the presence or absence of complications.

Yet another aspect of the present invention provides a kit for diagnosing coronavirus infection, which includes the above-described composition.

The "composition," "coronavirus infection" and "diagnosis" may be within the above-described ranges.

Yet another aspect of the present invention provides a method of introducing an antigen-coding gene of coronavirus, which includes transforming a subject with a gene encoding the spike antigen protein of coronavirus.

The "coronavirus," "spike antigen protein," and "transformation" may be within the above-described ranges.

In one embodiment, the introduction method may be applied with appropriate modifications by those skilled in the art, using methods that are well-known in the field.

Yet another aspect of the present invention provides a method of preventing or treating coronavirus infection, which includes administering the spike antigen protein of coronavirus to an individual in need thereof.

The "coronavirus," "spike antigen protein," "individual," "administration," "coronavirus infection," "prevention," and "treatment" may be within the above-described ranges.

The method may be administering the composition of the present invention in combination with a known composition or another pharmaceutical composition, which has a preventive or therapeutic effect on coronavirus infection, and the method may be simultaneous, separate, or sequential administration, or single or multiple administrations. Taking all of the above factors into consideration, it is important to administer a minimal amount that can achieve the maximum effect without side effects, and it can be easily determined by those of ordinary skill in the art.

Yet another aspect of the present invention provides a use of the spike antigen protein of coronavirus to prepare a drug for preventing or treating coronavirus infection.

The "coronavirus," "infection," "prevention," "treatment," and "spike antigen protein" may be within the above-described ranges.

### [Advantageous Effects]

It was confirmed that, in the spike antigen protein of coronavirus, according to one aspect, both protein cleavage sites present at the spike protein of coronavirus are modified, thereby inhibiting a cell membrane fusion ability and improving safety. In addition, when a vaccine using the antigen protein is administered, it can inhibit viral propagation by blocking the entry of coronavirus into cells by inducing the production of a large number of neutralizing antibodies, and thus can be used in various related industries/markets, including the prevention of coronavirus infection, alleviation and treatment of symptoms, and infection diagnosis.

### [Description of Drawings]

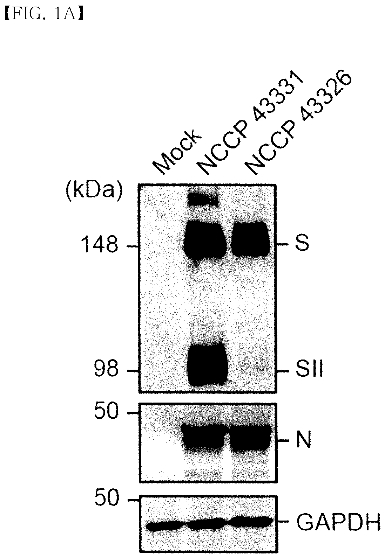

FIG. 1A shows the result of detecting a spike protein via SDD-PAGE followed by immunoblotting, using cell lysates obtained 48 hours after infection of cells with each of the two types of SARS-CoV-2 isolates NCCP43326 and NCCP43331 (provided by the National Pathogen Resource Bank of the Korean Disease Control and Prevention Agency), which had been subcultured twice in the Vero E6 cell line (N refers to the capsid protein, which is the structural protein of the virus).

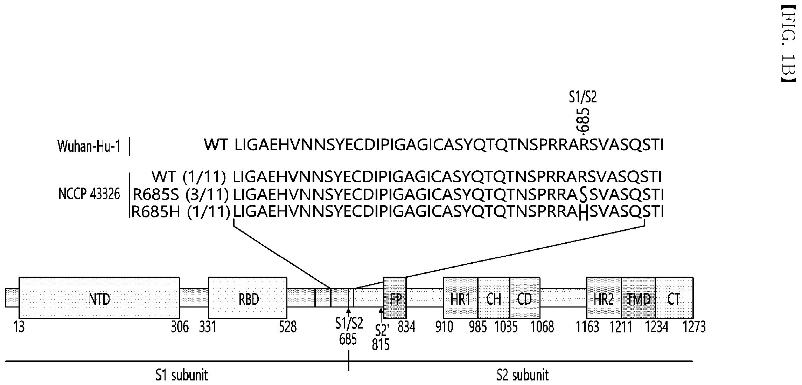

FIG. 1B is a schematic diagram of SARS-CoV-2 spike proteins, displaying its functional domains (NTD, N-terminal domain; RBD, receptor binding domain; S1/S2, S1/S2 furin cleavage site; S2', S2 priming site; FP, fusion peptide; HR, heptad repeat; CH, central helix; CD, connector domain; TMD, transmembrane domain; and CT, cytoplasmic tail). Specifically, the sequence shown in the schematic diagram represents the spike gene sequence confirmed by sequencing, which was into plasmid DNA after multiple passages of the SARS-CoV-2 isolate NCCP43326 in Vero E6. It shows that there are mutations in which the amino acid 685, which is the cleavage site of the furin protease, is substituted with serine or histidine from the original sequence, arginine.

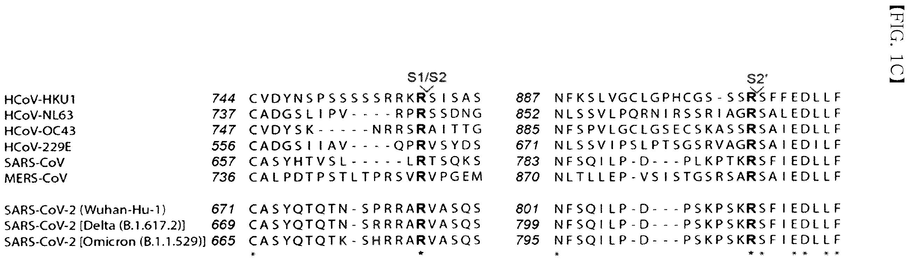

FIG. 1C shows that the S1/S2, which is the domain cleaved by recognition of the furin proteolytic enzyme, and S2', which is the domain recognized by the protease, during priming for fusion peptide exposure, exhibit the conservation of an amino acid sequence in various spike proteins of coronavirus.

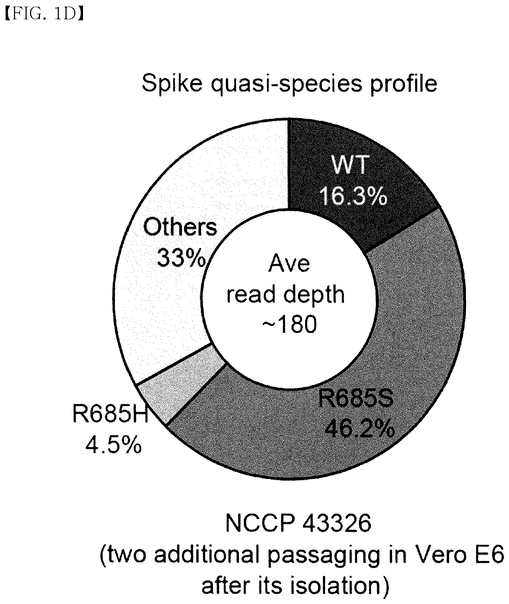

FIG. 1D shows the result of confirming the sequence confirmed by cloning a spike gene cloned from the SARS-CoV-2 isolate NCCP43326 after several passages in Vero E6 cells, as described in FIG. 1B through RNA sequencing. The sequencing results indicate that substitutions at the original amino acid residue R685, specifically to serine (R685S) or histidine (R685H), are predominantly present.

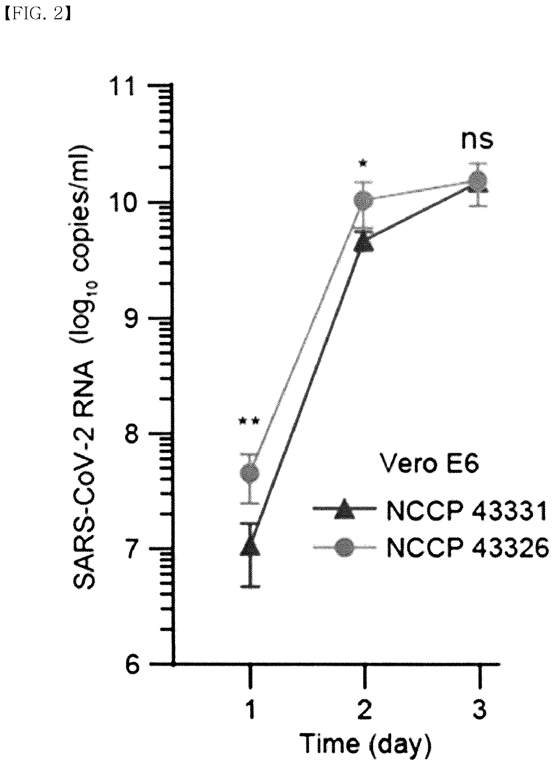

FIG. 2 shows the result of quantifying the gene copy number of each virus in the culture media over time after infecting the Vero E6 cell line with each of two SARS-CoV-2 isolates, NCCP43326 and NCCP43331, provided by the Korean Disease Control and Prevention Agency, at the same multiplicity of infection (MOI) (* indicates P <0.05, ** indicates P <0.01, and ns means no statistical significance).

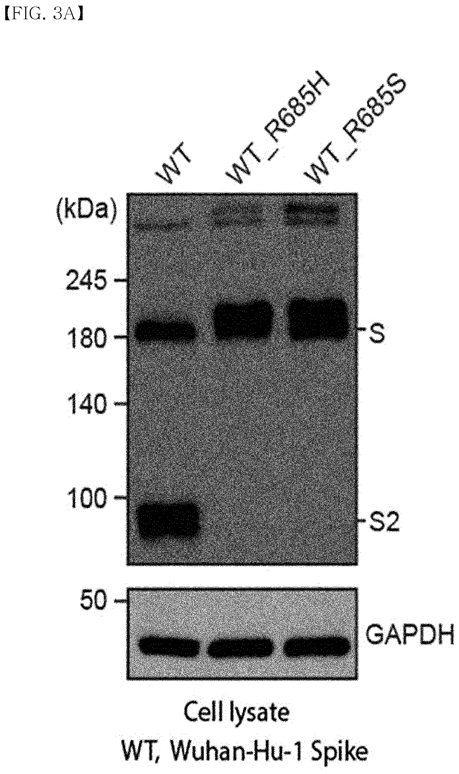

FIG. 3A shows the result of detecting the spike protein via SDD-PAGE followed by immunoblotting, using cell lysates prepared 48 hours after transfecting HEK293T cells with plasmid DNAs encoding three different types of spike proteins, as indicated.

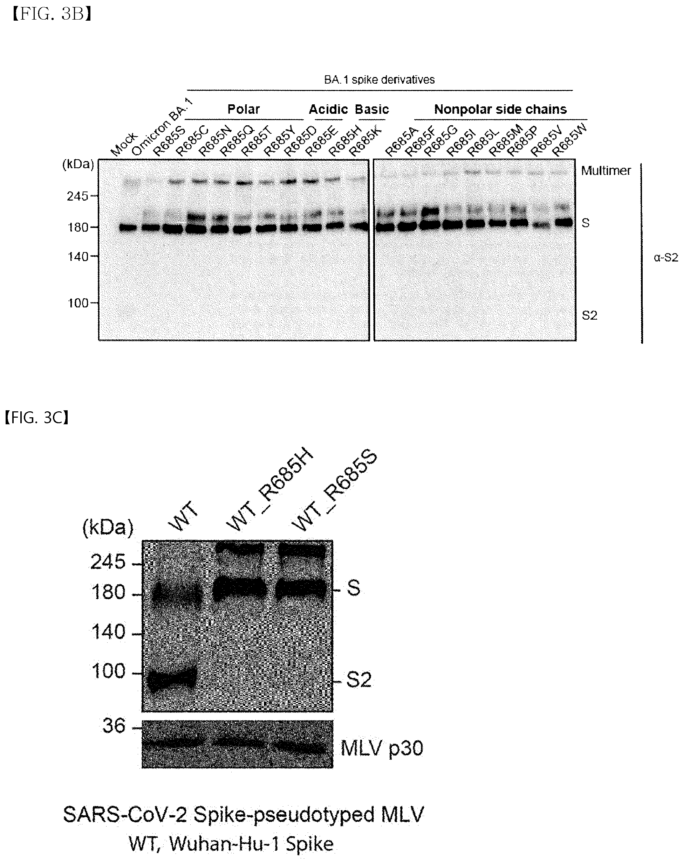

FIG. 3B shows the result of analyzing the cleavage pattern of the spike protein in which the arginine at position 685 (based on the Wuhan-Hu-1 reference sequence) of the Omicron BA.1 variant spike protein is substituted with the indicated amino acids. Specifically, it shows the results of detecting the full-length S and the S2 subunit that can be generated upon cleavage of the S protein, through immunoblotting using antibodies recognizing the C-terminus of the spike protein 48 hours after transfection of HEK293T cells with vectors expressing the indicated spike proteins. The results indicate that cleavage of the S protein into S1 and S2 subunits is blocked when the original amino acid residue R685 is replaced with any of 19 different types of amino acids, including the serine (R685S) or histidine (R685H).
FIG. 3C shows the result of detecting spike proteins via SDD-PAGE followed by immunoblotting, which is performed on the pseudotyped viruses released into the culture media after transfection of the HEK293T cell line with plasmid DNA encoding each of the three different types of spike proteins and a retrovirus packaging plasmid DNA.

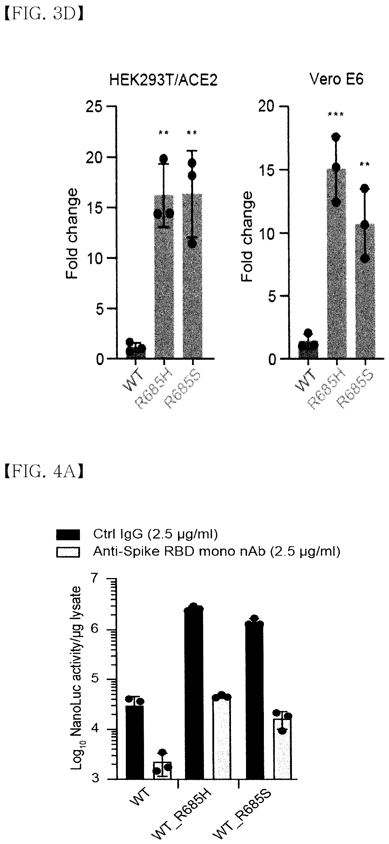

FIG. 3D shows the results of analyzing the cell entry efficiency of the pseudotyped viruses carrying each of the indicated three different types of SARS-CoV-2 spike proteins, prepared using recombinant retrovirus vectors (** indicates P <0.01, *** indicates P <0.001; unpaired two-tailed Student t-test).
FIG. 4A shows the results of analyzing the degrees of response of a modified spike protein with the indicated amino acid substitutions to a receptor-binding domain-targeting monoclonal antibody, using the pseudotyped viruses prepared as described in the present invention.

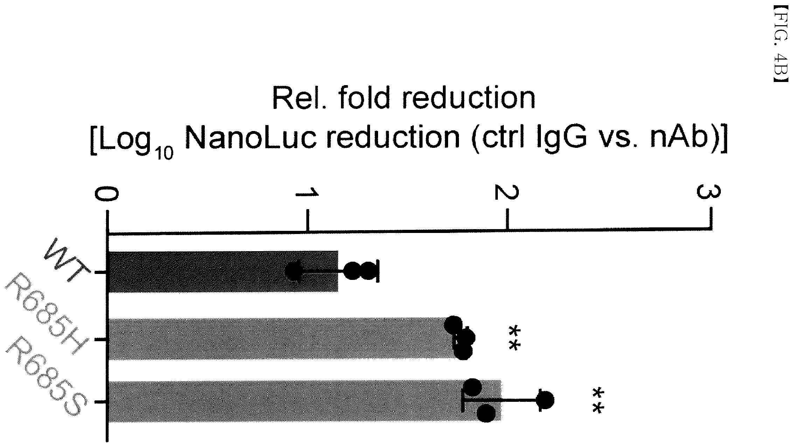

FIG. 4B shows the results of expressing the relative neutralizing efficacy of the single neutralizing antibody on a log₁₀ reduction scale, in comparison to that of a non-specific antibody used as a control, based on the results shown in FIG. 4A (** indicates P <0.01).

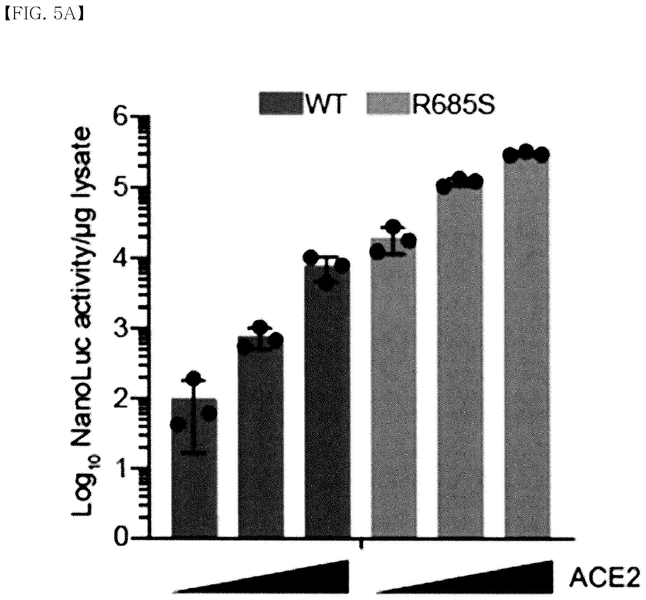

FIG. 5A shows the result of analyzing the entry efficiency of pseudotyped viruses carrying different SARS-CoV-2 spike proteins (WT and R685S) into the HEK 293T cell line expressing increasing levels of ACE2 protein, which is the receptor for SARS-CoV-2.

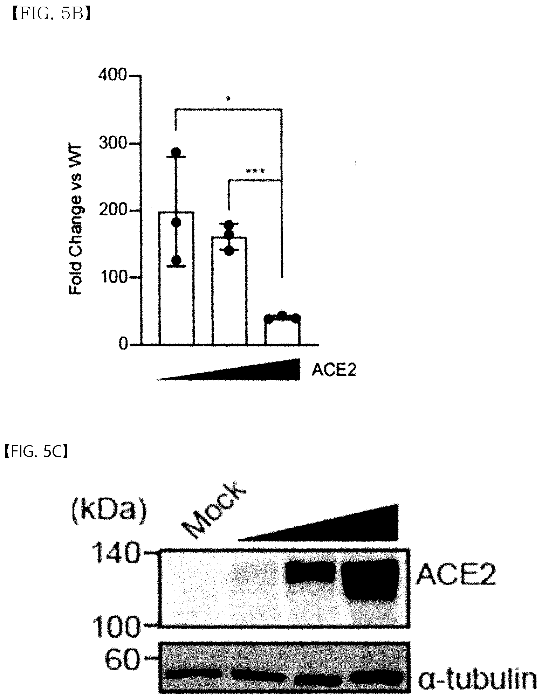

FIG. 5B shows the cell entry efficiency of pseudotyped viruses carrying the modified SARS-CoV-2 spike protein with an R685S substitution, compared to that of pseudotyped viruses with the Wuhan-Hu-01 isolate-derived spike protein, both of which were used in FIG. 5A (* indicates P <0.05, and *** indicates P <0.001).
FIG. 5C shows the result of confirming the level of the ACE2 receptor protein expressed in the HEK293 cell line in FIG. 5A.

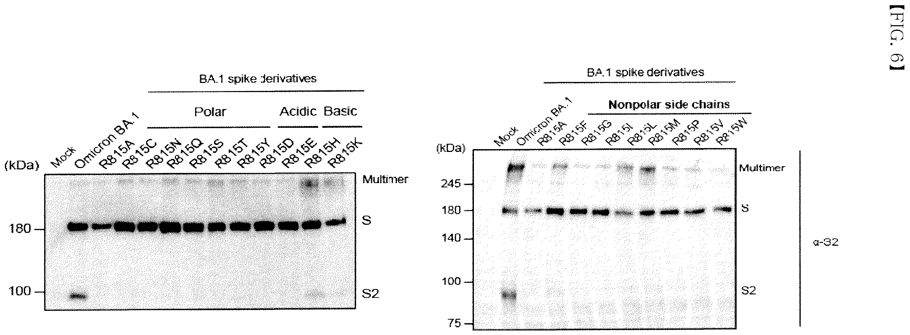

FIG. 6 shows the result of analyzing the cleavage pattern of a spike protein in which arginine at position 815 (based on the Wuhan-Hu-1 reference sequence) of BA.1 variant spike protein is substituted with indicated amino acids. Specifically, FIG. 6 shows the results of detecting the full-length S and the S2 subunit that can be produced by cleavage of the S, through immunoblotting using an antibody recognizing the C terminus of the spike protein 48 hours after transfecting HEK293 cells with a vector expressing the corresponding spike protein. The results indicate that S2 subunit generation is blocked when the original amino acid residue R815 is substituted with any one of 17 different amino acids except histidine (R815H) or lysine (R815K).

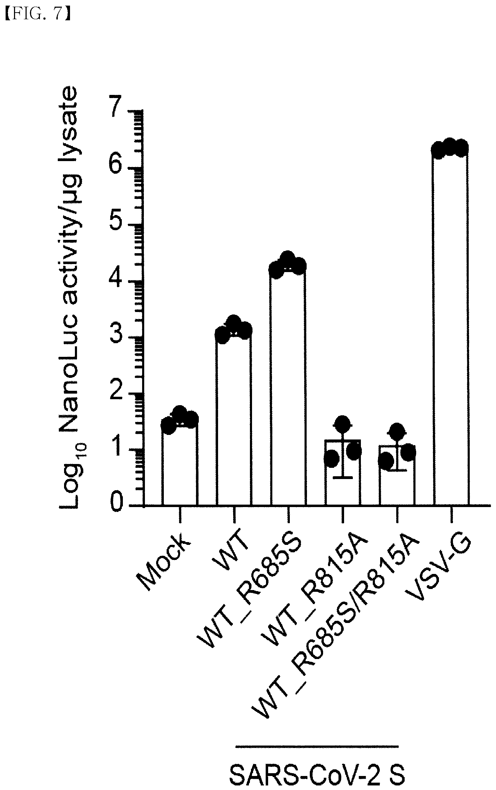

FIG. 7 shows the result of analyzing the effect of the substitution of an amino acid introduced to each modified spike protein on cell entry using a pseudotyped virus carrying each of the above-described SARS-CoV-2 spike proteins.

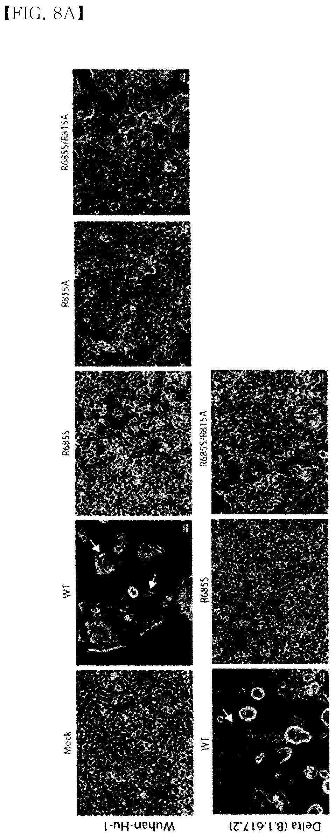

FIG. 8A shows the result of analyzing the cell membrane fusion activity of each spike protein after transfecting a plasmid expressing the SARS-CoV-2 Wuhan-Hu-01 spike or Delta variant (B.1.617.2) spike protein into HEK293T cells expressing ACE2 protein, which is a receptor for SARS-CoV-2, to overexpress the protein.

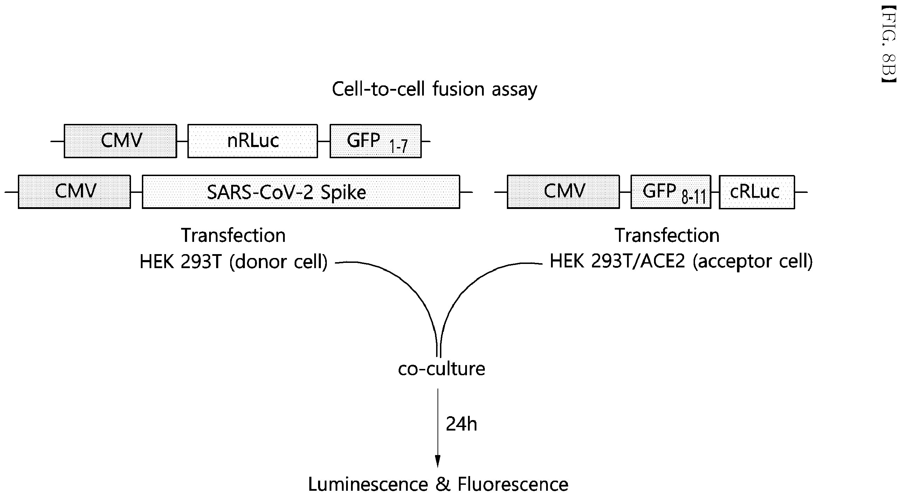

FIG. 8B is a schematic diagram that illustrates an experiment evaluating cell fusion activity using a dual split protein (DSP), which can quantify the cell membrane fusion activity of the spike protein (CMV, cytomegalovirus promoter; nRLuc, N-terminal end of the split *Renilla* luciferase; GFP1-7, N-terminal end of the split green fluorescence protein; GFP8-11, C-terminal end of the split green fluorescence protein; cRLuc, C-terminal end of the split *Renilla* luciferase).

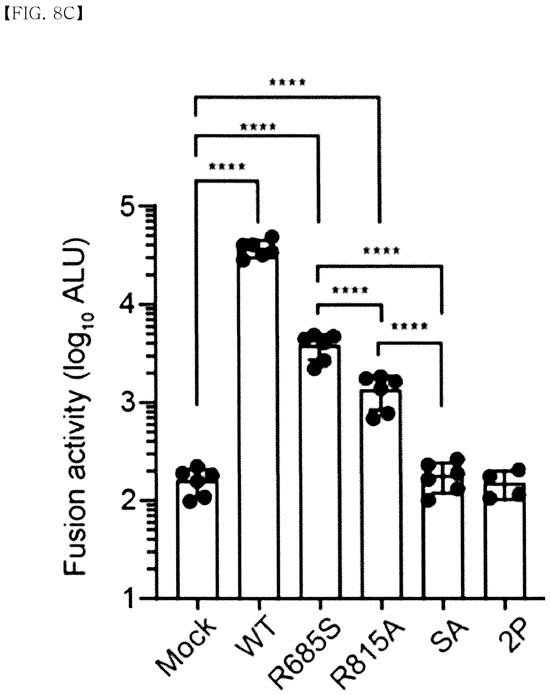

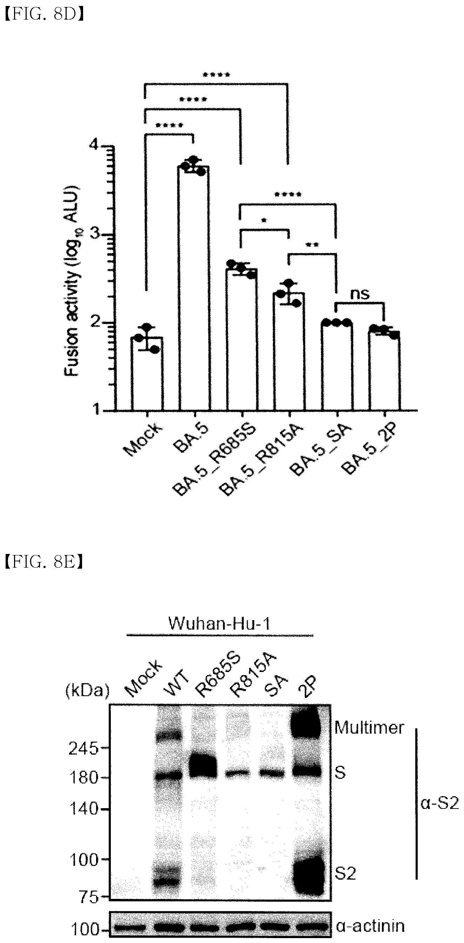

FIGS. 8C and 8D show the results obtained through an experiment evaluating cell fusion activity, as described in FIG. 8B, using cells that express vaccine antigen candidate spike proteins with multiple mutations introduced in the Wuhan-Hu-01 and Omicron BA.5 (B.1.1.529.5) sequence-based spike proteins, respectively (* indicates P <0.01; ** indicates P <0.05; **** indicates P <0.0001; ns, not significant (unpaired two-tailed Student t-test)).

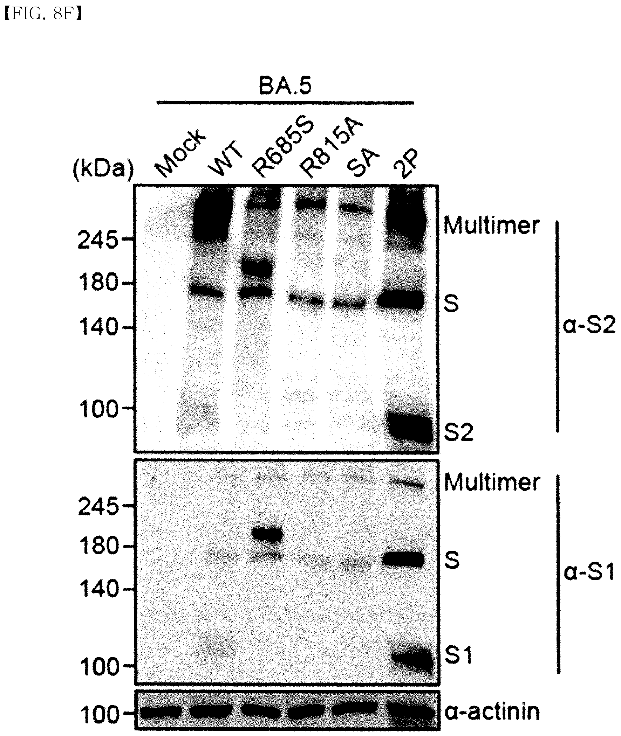

FIGS. 8E and 8F show the results of detecting spike proteins via SDD-PAGE followed by immunoblotting, performed using cell lysates obtained 48 hours after transfection with plasmid DNAs expressing each of the vaccine antigen candidate spike proteins with multiple mutations introduced in the Wuhan-Hu-01 and Omicron BA.5 (B.1.1.529.5) sequence-based spike proteins, respectively.

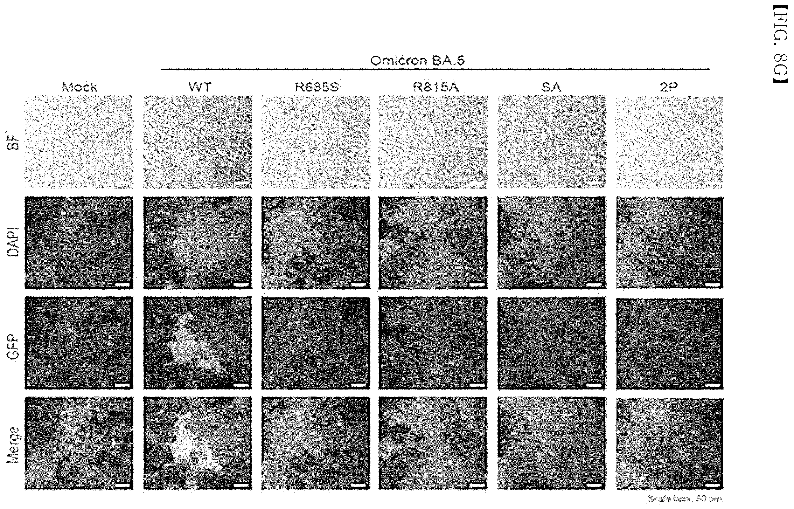

FIG. 8G shows the results of analyzing fused cells through a fluorescence microscope after conducting the experiment evaluating the cell fusion activity as described in FIG. 8B using cells that express a vaccine antigen candidate spike protein with multiple mutations introduced in the Omicron BA.5 (B.1.1.529.5) sequence-based spike protein (BF, bright field; DAPI, 4',6-diamidino-2-phenylindole; GFP, green fluorescence protein, DAPI is used to stain the nuclei in cells).

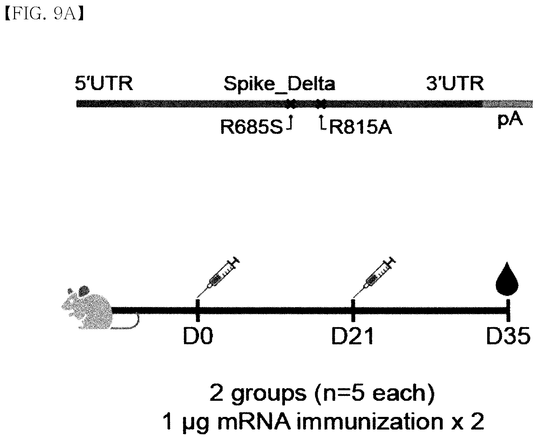

FIG. 9A is a schematic diagram illustrating an experimental schedule and mRNA, which was used to test the immunogenicity of a mutated spike protein through intramuscular injection (twice on D0 and D21 with a 3-week interval) of LNP-formulated mRNA that encodes SARS-CoV-2 Delta variant (B.1.617.2) spike protein (Delta_SA) in which cell membrane fusion caused by spike protein cleavage and spike protein expression is blocked by introducing the substations of R685S and R815A (hereinafter, the substitution with these two amino acids is represented as SA) into mice (BALB/c) (5'UTR, 3'-untranslated region; 3'UTR, 3'-untranslated region; pA, poly(A) tail). The mRNA was synthesized using pseudouridine, and the 5'-terminus has a cap structure.

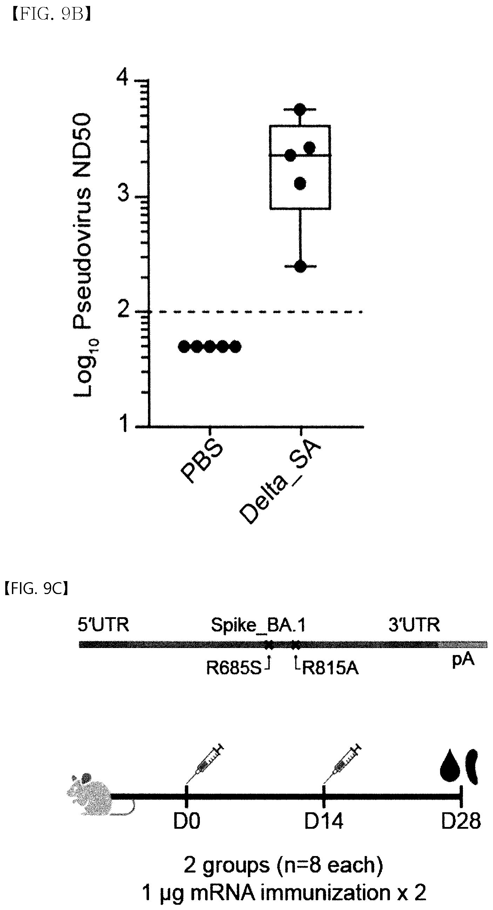

FIG. 9B shows the results of analyzing the neutralizing antibody levels in sera using pseudotyped viruses two weeks (week 5) following intramuscular administration of an mRNA-LNP vaccine encoding the Delta variant (B.1.617.2) spike protein. The vaccine was administered twice with a 3-week interval, as per the animal experiment schedule outlined in FIG. 9A. The neutralizing dilution 50 (ND₅₀) value is defined as the reciprocal serum dilution fold showing 50% neutralization efficiency.
FIG. 9C is a schematic diagram illustrating an experimental schedule and mRNA, which was used to test the immunogenicity of a mutated spike protein through intramuscular injection (twice on D0 and D21 with a 3-week interval) of LNP-formulated mRNA that encodes SARS-CoV-2 omicron BA.1 (B.1.1.529) variant spike protein (BA.1_SA), where spike protein cleavage and cell membrane fusion are blocked, into BALB/c mice. The mRNA was synthesized using pseudouridine, and the 5'-terminus has a cap structure.

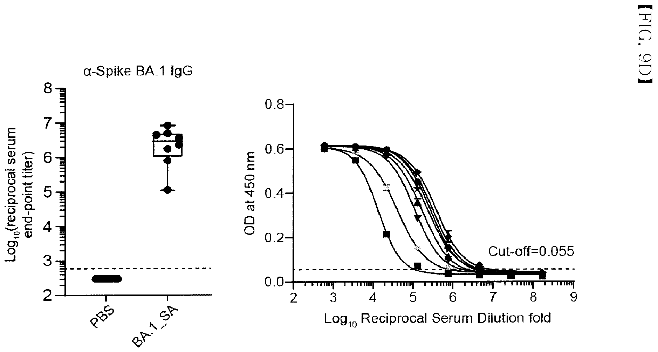

FIG. 9D shows the results analyzing BA.1 spike protein-binding antibody levels in sera by enzyme-linked immunosorbent assay (ELISA) two weeks (week 4) after two intramuscular injections with an mRNA-LNP vaccine encoding the Omicron BA.1 (B.1.1.529) variant spike protein with a 2-week interval, as described in FIG. 9C. Specifically, the left panel presents the endpoint titers of binding antibodies in sera, calculated based on the cut-off value determined in the right panel, which depicts the cut-off value of absorbance based on the ELISA results for each dilution of sera from eight experimental mice in the vaccine group.

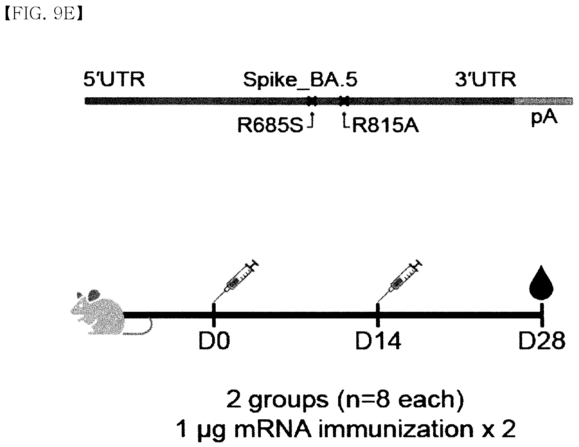

FIG. 9E is a schematic diagram illustrating an experimental schedule and mRNA used to test the immunogenicity of LNP-formulated mRNA encoding SARS-CoV-2 SARS-CoV-2 Omicron variants BA.5 (B.1.1.529.5) modified spike antigen protein (BA.5_SA), in which spike protein cleavage and cell membrane fusion are blocked. The vaccine was administered by intramuscular injection into BALB/c mice twice (on D0 and D21) with a 2-week interval. The mRNA was synthesized using an unmodified UTP or modified nucleic acid, pseudouridine triphosphate (ψUTP), and the 5'-terminus has a cap structure.

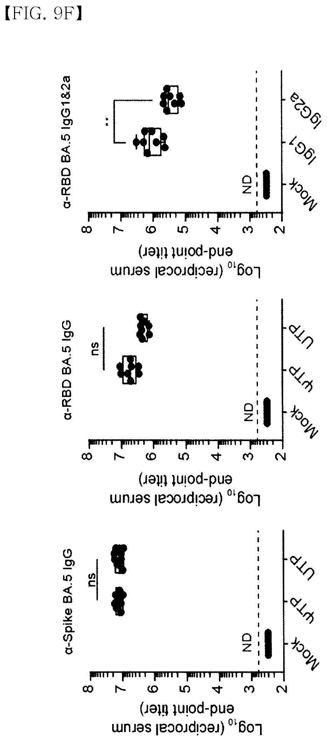

FIG. 9F shows the results of analyzing BA.5 spike protein-binding antibody levels in sera by ELISA two weeks (week 4) after the booster vaccination, as described in FIG. 9E. Specifically, the graphs show the total IgG endpoint titers for full-length spike protein-binding antibodies, the total IgG endpoint titer for receptor-binding domain (RBD)-binding antibodies, and the endpoint titers for RBD-binding antibodies, IgG1 and IgG2a isotypes, in order from left to right.

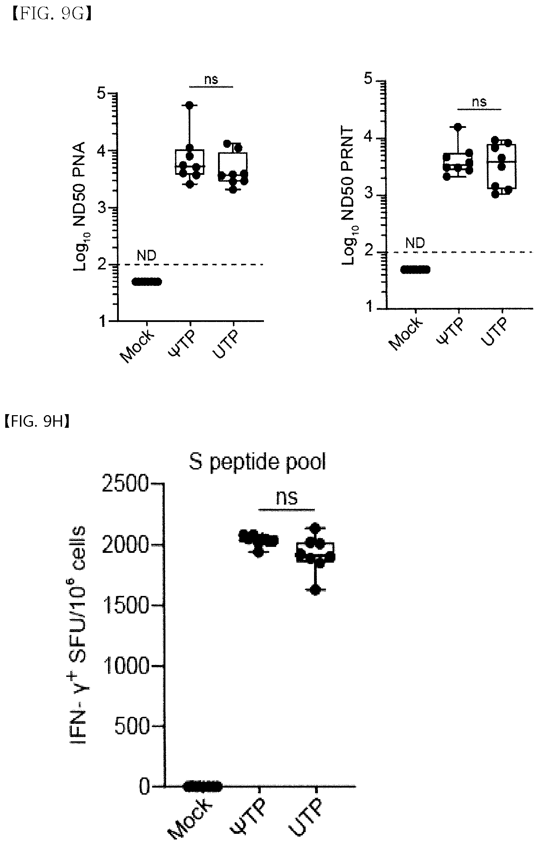

FIG. 9G shows the results of analyzing the neutralizing antibody titers in sera using pseudotyped viruses and live viruses (BA.5 Omicron variants) two weeks (week 4) after the booster vaccination, as described in FIG. 9E (ND₅₀: neutralizing dilution 50, PNA: pseudovirus neutralization assay, PRNT: plaque reduction neutralization test, ND: not detected, ns: not significant (unpaired two-tailed Student t-test, and the dotted line represents the limit of detection (LOD)).
FIG. 9H shows the result of analyzing antigen-specific T cell immunogenicity through an enzyme-linked immunospot (ELISpot) assay using splenocytes isolated from the spleen of mice two weeks (week 4) after receiving a booster vaccination, as described in FIG. 9E (SFU: spot forming unit, ns: not significant (unpaired two-tailed Student t-test)).

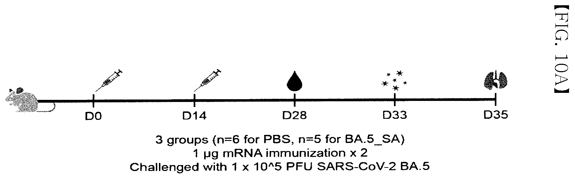

FIG. 10A is a schematic diagram illustrating the experimental schedule used to confirm the protective immune responses of the vaccine, after intramuscular injection of the LNP-formulated mRNA encoding the SARS-CoV-2 Omicron BA.5 (B.1.1.529.5) variant spike protein (BA.5_SA), in which spike protein cleavage and cell membrane fusion are blocked, into BALB/c mice, as described in FIG. 9E.

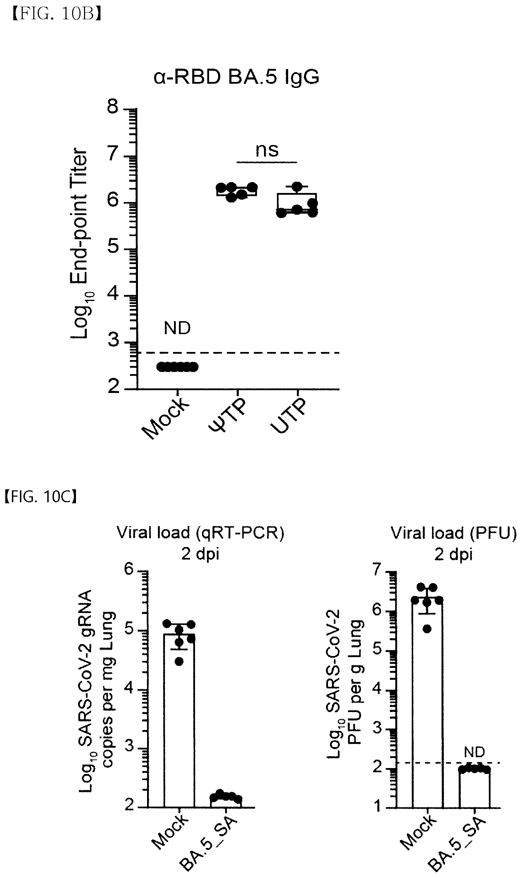

FIG. 10B shows the result of analyzing a BA.5 spike RBD-binding antibody levels in sera by ELISA, conducted two weeks (week 4) after the booster vaccination, which followed the first intramuscular injection of the mRNA-LNP vaccine encoding the omicron BA.5 (B.1.1.529.5) variant spike protein, as described in FIG. 10A (ND: not detected, ns: not significant (unpaired two-tailed Student t-test, and the dotted line represents the limit of detection (LOD)).
FIG. 10C shows the results of the experiments evaluating the protective efficacy of the immunized mice upon challenge infection with Omicron BA.5 (B.1.1.529.5) variant (1×10⁵ PFU) via the nasal cavity on day 19 (D33). The mice were immunized with two intramuscular injections of the mRNA-LNP vaccine encoding the Omicron BA.5 (B.1.1.529.5) variant spike protein, administered two weeks apart, as described in the experimental schedule in FIG. 10A (ND: not detected, and the dotted line represents the limit of detection (LOD)).

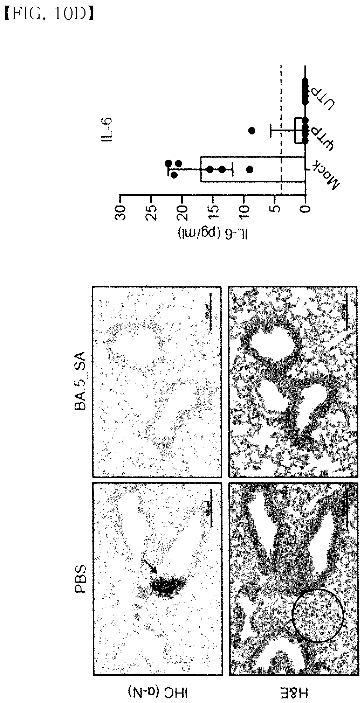

FIG. 10D shows the detection of viral capsid protein in the lungs of infected mice, collected on day 2 post-infection, through immunohistochemistry (IHC) in the mouse experiment described in FIG. 10A. (In the IHC results, viral capsid protein is indicated by arrows. Hematoxylin and eosin (H&E) staining shows damaged alveolar structures marked by circles.) Specifically, the right panel shows the results of analysis of the inflammatory cytokine IL-6 induced by infection through ELISA after homogenizing the lung of an experimental mouse harvested on day 2 of infection (the dotted line represents the limit of detection (LOD)).

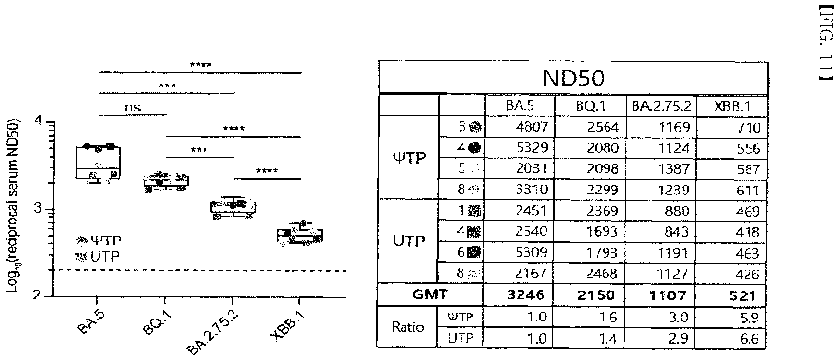

FIG. 11 shows the results of analyzing the neutralizing antibody titers in sera using various Omicron sub-variant spike-pseudotyped viruses, two weeks (week 4) after booster immunization. The mice were given two intramuscular injections of the mRNA-LNP vaccine encoding the omicron BA.5 (B.1.1.529.5) variant spike protein, administered two weeks apart, as described in FIG. 9E (ND₅₀: neutralizing dilution 50). Specifically, the right panel presents a table showing the ND₅₀ values (*** indicates P <0.001; **** indicates P <0.0001; ns indicates not significant (unpaired two-tailed Student t-test), and the dotted line represents the limit of detection (LOD)). Here, data points for the mRNA vaccines prepared using ψUTP and UTP are shown in different colors (GMT: geometric mean titer, Ratio: relative neutralization efficacy of neutralizing antibodies induced by immunization with mRNA vaccines prepared using ψUTP and UTP against the indicated variants, in comparison with the neutralization efficacy toward the BA.5 variant).

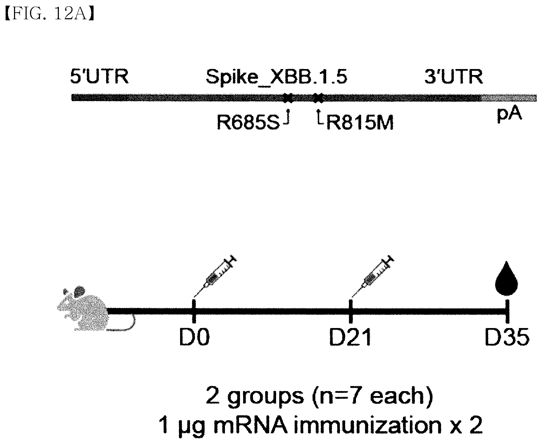

FIG. 12A is a schematic diagram illustrating the experimental schedule for evaluating immunogenicity through intramuscular injection (on D0 and D21, with a 3-week interval) of LNP-formulated mRNA encoding SARS-CoV-2 Omicron sub-variant XBB.1.5's modified spike antigen protein (XBB.1.5_SM), in which spike protein cleavage and cell membrane fusion function were blocked by introducing R685S and R815M substitutions, into mice (BALB/c). The mRNA is synthesized using unmodified UTP, and the 5'-terminus has a cap structure.

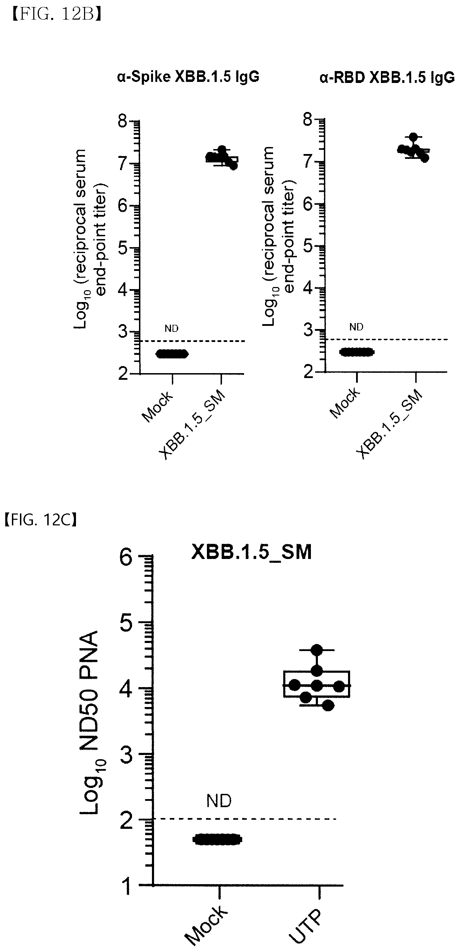

FIG. 12B shows the results of analyzing XBB.1.5 spike protein-binding antibody levels in sera via ELISA two weeks (week 4) after secondary vaccination, as described in FIG. 12A. Specifically, the graphs show the total IgG endpoint titers for full-length spike protein-binding antibodies, and the total IgG endpoint titers for receptor-binding domain (RBD)-binding antibodies, presented from left to right.
FIG. 12C shows the result of analyzing neutralizing antibody titers in sera using pseudotyped viruses two weeks (week 4) after the vaccination as described in FIG. 12A (ND₅₀: neutralizing dilution 50, PNA: pseudovirus neutralization assay, ND: not detected, and the dotted line represents the limit of detection (LOD)).

### [Modes of the Invention]

Hereinafter, the present invention will be described with reference to examples. However, these examples are merely provided to illustrate the present invention, and the scope of the present invention is not limited thereto.

### Examples

### Examples

### 1. Identification of Vero E6 cell line-adapted mutations in SARS-CoV-2

Two SARS-CoV-2 isolates (NCCP43326 and NCCP43331) provided by the National Pathogen Resource Bank of the Korea Centers for Disease Control and Prevention were passaged in the Vero E6 cell line, which is a Savannah monkey kidney-derived cell line, and the cleavage pattern of spike proteins was assessed.

A serial passage experiment was performed by infecting Vero E6 cells (1×10⁵ cells per well in a 6-well plate) with SARS-CoV-2 at a multiplicity of infection (MOI) of 0.01. Three days post-infection, the viruses were collected and used to infect new Vero E6 cells (1×10⁵ cells/6-well-plate well) at the same MOI. After two passages (referred to as P2), infected cells were lysed, and the cell lysates were subjected to SDD-PAGE followed by immunoblotting with an antibody targeting the C-terminus of the SARS-CoV-2 spike protein. The results showed a different spike protein cleavage pattern exclusively in the cell lysates derived from cells infected with the NCCP43326 virus (FIG. 1A).

In addition, during serial passages of the NCCP43326 viruses in Vero E6 cells, viral RNA was extracted from the culture media containing SARS-CoV-2. cDNA representing the spike-coding gene was synthesized via reverse transcription, followed by PCR amplification. The amplified cDNA was cloned into a plasmid for sequence analysis. Through analyzing 11 independent clones, two representative mutations, resulting in R685H or R685S amino acid substitutions at the furin cleavage site, were identified (FIG. 1B). Notably, no spike protein genes with two or more double mutations at or near amino acid positions 685 and 815 in any of the 11 clones analyzed. This indicates that variants with these mutations were generated individually into the viral spike gene (FIG. 1C).

In addition, the RNA extracted from the passaged NCCP43326 viruses was analyzed using next-generation sequencing, with positional read depths set at approximately 180. The results revealed various mutations in the spike-coding sequence, with more than 50% of the sequences at amino acid position 685 exhibiting substitutions to either serine (Ser) or histidine (His) (FIG. 1D).

### 2. Confirmation of the function of spike proteins with a mutation at the furin cleavage site

To evaluate the impact of furin cleavage site mutations on viral properties, growth kinetics were analyzed using NCCP43326 and NCCP43336 P2 virus stocks.

Vero E6 cells (1×10⁵ cells/6-well-plate well) were infected with each virus at an MOI of 0.01, and viral RNA gene copy numbers in the culture medium were measured at 24, 48, and 72 hours post-infection by quantitative reverse transcription PCR (qRT-PCR). The results showed that the NCCP43326 isolate, which mainly produces the uncleaved spike protein, exhibited higher levels of virus production and secretion at 24 and 48 hours post-infection compared to the NCCP43331, which expresses the truncated spike protein (FIG. 2).

### 3. Evaluation of the function of mutations at amino acid position 685 of the SARS-CoV-2 spike protein

To analyze the functional changes resulting from the substitution of arginine at position 685 in the SARS-CoV-2 spike protein with histidine or serine, a pseudotyped virus displaying the SARS-CoV-2 spike protein on its surface was generated to evaluate the effect of these mutations on host cell entry.

The pseudotyped virus was produced by co-expressing the SARS-CoV-2 spike protein with the packaging proteins of murine leukemia virus (MLV), a retrovirus, leading to the generation of a retrovirus particle pseudotyped with the SARS-CoV-2 spike protein. The pseudotyped virus mimics SARS-CoV-2 in its cell entry mechanism and antigenicity, making it useful for studying the virus entry process as a substitute for the highly pathogenic SARS-CoV-2.

Prior to producing the pseudotyped virus with the spike protein in which the amino acid at position 685 was mutated, the impact of each mutation on the spike protein cleavage pattern was analyzed. HEK293T cells were transfected with plasmid DNA encoding these mutant spike proteins, and the cleavage pattern was assessed through immunoblotting using an antibody specific to the C-terminus of the SARS-CoV-2 spike protein. The analysis revealed that mutations substituting arginine at position 685 with serine (Ser, R685S) or histidine (His, R685H) blocked the cleavage of the spike protein into S1 and S2 subunits (FIG. 3A).

In addition, vectors expressing modified spikes, in which the arginine residue at position 685 of Omicron BA.1 (based on the Wuhan-Hu-1 reference sequence) was substituted with each of 19 types of different amino acids, were generated to analyze the cleavage patterns of these modified spikes by proteolytic enzymes, including furin, in the HEK293T cell line. Through immunoblotting using an antibody specifically recognizing the C-terminus of the SARS-CoV-2 spike protein, it was confirmed that the production of the S2 subunit was inhibited in modified spike antigens when the amino acid at position 685 was replaced with any amino acids other than arginine (FIG. 3B).

Afterward, to verify whether the pseudotyped virus particles reflected the cleavage patterns of spike proteins observed in packaging cells, pseudotyped viruses were produced by the above-described method, lysed, and the spike proteins were analyzed by immunoblotting. The results confirmed that the pseudotyped viral particles contained uncleaved spike proteins, consistent with the cleavage pattern observed in the packaging cells (FIG. 3C).

Then, the cell entry efficiency of the pseudotyped viruses was evaluated in the HEK293T/ACE2 cell line and the Vero E6 cell line, which express a receptor for SARS-CoV-2. The cell entry efficiency was analyzed by measuring the activity of nanoluciferase. This enzyme is expressed from mRNA transcribed following the transfer of the luciferase gene-encoding retroviral genome packaged within the pseudotyped virus. This allows for the measurement of viral entry efficiency through luciferase activity. The analysis results confirmed that the pseudotyped virus loaded with the spike protein with a mutation at position 685 exhibited approximately 15-fold higher cell entry efficiency compared to the wild-type spike protein (FIG. 3D). Such enhanced entry efficiency suggests that specific amino acid substitutions preventing furin cleavage may induce structural changes that expose more receptor-binding domains in the spike trimer. In addition, these modified spike proteins show a superior ability to bind to receptors compared to the wild-type spike protein in both HEK293T/ACE2 and the Vero E6 cell lines, which express the ACE2 receptor.

### 4. Evaluation of the potential for additional exposure of the receptor-binding domain through furin enzyme cleavage-blocking mutations

To determine whether the receptor-binding domain is additionally exposed due to the structural change in the spike protein caused by mutations introduced at amino acid position 685, the reactivity to a monoclonal neutralizing antibody, which specifically recognizes a receptor-binding domain, was compared using the pseudotyped viruses generated in the above example.

As a result, compared to the wild-type, it was confirmed that the pseudotyped virus loaded with a spike protein in which protein cleavage was blocked reacted more sensitively to the neutralizing antibody, thereby inhibiting the entry of the virus (FIGS. 4A and 4B). When using an amount of RBD-recognition antibody at a final concentration of 2.5 µg/mL, which inhibits the entry of wild-type spike protein pseudotyped viruses into the HEK293T/ACE2 cell line by 1log₁₀, it was confirmed that the pseudotype virus loaded with the furin-cleavage-resistant mutant spike proteins could form a structure that exposes the RBD more effectively. This increased exposure enhanced the RBD-recognition antibody's ability to inhibit ACE receptor-mediated cellular entry, resulting in an approximately 1log₁₀-fold greater reduction in viral entry compared to the pseudotype virus with the wild-type spike protein (FIG. 4B).

### 5. Evaluation of changes in receptor-binding ability induced by a furin cleavage-blocking mutation in the spike protein

To evaluate the impact of enhanced receptor-binding domain (RBD) exposure, caused by a mutation at arginine 685 in the spike protein, on viral entry efficiency, two types of pseudotyped viruses were prepared: one containing the wild-type spike protein from Wuhan-Hu-01 and the other harboring the R685S mutant spike protein. These pseudotyped viruses were used to infect HEK293T cells that expressed increasing levels of the ACE2 receptor. HEK293T cells were transfected with a DNA plasmid encoding the ACE2 receptor at concentrations of 0.1 µg, 1 µg, and 10 µg to achieve increasing levels of ACE2 expression. Following transfection, the cells were infected with pseudotyped viruses carrying either the wild-type or the R685S mutant spike protein, and viral entry efficiency was evaluated. The results indicated that, at the lowest ACE2 receptor expression levels, the pseudotyped virus containing the R685S mutant spike protein demonstrated approximately 200-fold higher viral entry efficiency compared to the virus with the wild-type spike protein. However, under conditions of maximal ACE2 receptor expression, this enhancement effect was reduced, with the R685S mutant exhibiting only a 10-fold increase in entry efficiency (FIGS. 5A and 5B). Immunoblotting analysis confirmed a dose-dependent increase in ACE2 protein levels with increasing amounts of transfected ACE2 expression vector (FIG. 5C).

### 6. Identification of amino acid residues at position 815 that inhibit spike protein cleavage

After confirming that cleavage at the S1/S2 site was inhibited by the amino acid substitution at the Omicron BA.1 spike protein S1/S2 cleavage site in Example 3, the effect of substituting the amino acid at position 815 (based on the Wuhan-Hu-1 reference sequence), which is present at an S2' cleavage site that can be cleaved by a protease other than furin, was analyzed. An amino acid group that affects spike protein cleavage was determined by substituting the existing arginine with each of 19 different amino acids.

Specifically, a series of vectors expressing the modified spikes, in which the arginine residue at position 815 of the Omicron BA.1 spike protein (based on the Wuhan-Hu-1 reference sequence) was substituted with each of the 19 different amino acids, were prepared to analyze the pattern of cleaving the modified spikes by an intracellular protease, including furin in the HEK293T cell line, thereby determining which amino acid group affects spike protein cleavage. As a result of detecting the spike proteins through immunoblotting using antibodies specifically recognizing the C-terminus of the SARS-CoV-2 spike protein, it was confirmed that the production of the S2 subunit was inhibited in the modified spike antigens with substitutions for arginine, except when the new residue was histidine or lysine (FIG. 6).

### 7. Analysis of functional changes in spike protein by a mutation at the cleavage target amino acid at position 815

It was confirmed that the above-described amino acid substitutions found at the amino acid position 685, as described in Examples 4 and 5, block cleavage during the spike protein production while maintaining the ability to bind to the ACE2 receptor and facilitating the entry of the pseudotyped virus into the cells. The findings from the above examples suggest that cleavage at other sites of these non-cleaved spike proteins can lead to cell fusion at the cell membrane or endosomal membrane, subsequently releasing the pseudotyped viral genome. Furthermore, Example 6 demonstrated that substitution of a specific amino acid at the S2' cleavage site influences spike protein cleavage. Therefore, the present inventors analyzed the impact on virus entry by substituting the arginine at position 815, which is targeted by an additional spike protein cleavage enzyme, with alanine.

For the R815A-modified spike, where the R815 residue located at the S2' cleavage site (position 815) was substituted with alanine, it was found that the ability of the pseudotyped virus to enter cells was completely compromised. In the above example, it was confirmed that the intracellular entry of the pseudotyped virus, which was improved when the amino acid at position 685 was mutated, was blocked when the R815A mutation was introduced at amino acid 815 (FIG. 7).

### 8. Comparative evaluation of cell membrane fusion ability of modified spike protein

The capability of a modified vaccine antigen candidate spike protein to induce cell membrane fusion was evaluated, specifically where amino acid 685 at the S1/S2 cleavage site was substituted with serine, and amino acid 815 at the S2' cleavage site was substituted with alanine. When the wild-type (WT) spike proteins (Wuhan-Hu-1 lineage and Delta variant spike protein) were co-expressed together with the ACE2 protein as a receptor, binding of these two proteins led to cell membrane fusion and the formation of syncytia (FIG. 8A). On the other hand, substituting the R685 amino acid, which is the S1/S2 cleavage site, with serine resulted in a significant loss of cell membrane fusion activity, with no multinucleated giant cells generated (FIG. 8A). In addition, the R815A substitution was shown to completely prevented cell membrane fusion by blocking the exposure of the fusion peptide. When these amino acid substitutions were introduced into the Delta (B.1.617.2) variant spike protein, a similar inhibition of cell fusion was observed (FIG. 8A, the lower panel).

Further, the cell membrane fusion capability of each spike protein was evaluated through a dual split protein (DSP) assay, as illustrated in FIG. 8B. The visual evaluations using a microscope shown in FIG. 8A indicated that the modified spike protein with serine and alanine substitutions at residues 685 and 815, respectively, exhibited significantly inhibited cell fusion, preventing the formation of multinucleated giant cells. In the DSP assay, modified spike proteins (R685S and R815A) with a single amino acid substitution displayed a lower cell fusion ability compared to the wild-type, but higher than that of the negative control (FIG. 8C). Notably, the modified spike protein with both substitutions (SA) demonstrated a complete loss of cell membrane fusion ability. Additionally, a spike protein antigen (2P) containing proline substitutions at positions 986 and 987, which is used in current mRNA vaccines, was shown to maintain a prefusion conformation and did not induce cell fusion (FIG. 8C).

The cell fusion ability of these modified proteins followed a similar pattern in experiments using spike proteins based on the Omicron BA.5 variant sequence (FIG. 8D).

The cleavage patterns of the above-described modified spikes with one or two amino acid substitutions, alongside both Wuhan-Hu-1 and Omicron BA.5-derived wild-type (WT) spike proteins used for comparison, were analyzed. It was confirmed that SA substitution introduction inhibits cleavage at the S1/S2 site by analyzing cell lysates through immunoblotting 48 hours after transfecting the HEK293T cell line with plasmids expressing these spike antigens (FIG. 8E). On the other hand, it was seen that the spike antigen with the 2P substitution, expressing a prefusion conformational spike, generated a substantial amount of S2 domain (FIG. 8E). Similar results were also observed with the Omicron BA.5 variant spikes (FIGS. 8F and 8G). The cleavage of spike protein can lead to the extracellular release of the S1 domain, which can trigger an inflammatory response via the stimulation of a receptor inducing an innate immune response, called toll-like receptor 4 (TLR4). Unlike the "2P design," the modified spike protein with the "SA" substitution was confirmed to prevent the separation of the S1 and S2 domains, highlighting its potential as a safe vaccine antigen.

### 9. Evaluation of immunogenicity of a modified spike antigen with inhibited spike cleavage

To confirm the immunogenicity of a double-mutated spike protein, in which both amino acids 685 and 815 were mutated, we synthesized spike protein-expressing mRNA that has a vaccine antigen-designing sequence. After its encapsulation with lipid nanoparticles (LNPs), the resulting mRNA-LNP vaccine was administered intramuscularly into mice to measure neutralizing antibody titers.

To produce an mRNA-LNP vaccine, mRNAs that encode spike protein sequences that have the double mutations incorporated in the spike proteins of B.1.617.2 Delta variant, B.1.1.529 Omicron BA.1 variant, B.1.1.529.5 Omicron BA.5 variant (FIG. 9A, FIG. 9C, and FIG. 9E) were produced via *in vitro* transcription (IVT). During IVT, a modified nucleotide, pseudouridine triphosphate (ψUTP), was incorporated to mitigate innate immune activation that can be triggered by foreign mRNA. For the B.1.1.529.5 Omicron BA.5 variant, mRNAs were synthesized using either ψUTP or unmodified uridine triphosphate (UTP) to compare their immunogenicity. During mRNA IVT, the CleanCap Reagent AG [(3'OMe) m7(3'OMeG)(5')ppp(5')(2'OMeA)pG] from TriLink was used for 5' capping to enhance protein expression.

The mRNA-LNPs were formulated in a microfluidic system by combining a lipid solution containing cholesterol, polyethylene glycol-conjugated lipid (ALC-0159), an ionizable lipid (ALC-0315), and distearoylphosphatidylcholine (DSPC) with the mRNA.

After quantifying the formulated mRNA levels using RiboGreen absorbance measurement, Delta variant-based mRNA-lipid nanoparticle (LNP) vaccine was intramuscularly injected into the hind limb muscles of BALB/c mice (5 mice per group) at 1 µg per dose, followed by a booster three weeks later (FIG. 9A). Omicron BA.1 variant-targeting mRNA-LNP vaccine was similarly administered every two weeks at the same dosage (8 mice per group) (FIG. 9C). BA.5 variant-targeting mRNA-LNP vaccine was also similarly administered every two weeks at the same dosage (8 mice per group) (FIG. 9E).

To evaluate the immunogenicity of the modified spike protein, serum was isolated two weeks post-second vaccination. Strong spike protein-binding antibody responses induced by the BA.1 vaccine was verified through enzyme-linked immunosorbent assay (ELISA) (FIG. 9D). Furthermore, both BA.5 mRNA vaccines, produced using either ΨUTP or UTP, elicited comparable high endpoint binding antibody titers (FIG. 9F). Further analysis revealed balanced receptor-binding domain (RBD)-binding IgG2a and IgG1 antibody responses, indicating activation of both Th1 and Th2 pathways in mice receiving the ψUTP-incorporated vaccine (the right panel of FIG. 9F). Neutralizing antibody titration assays demonstrated that all vaccinated groups had ND50 values exceeding 1,000, substantially higher than the negative controls (FIGS. 9B and 9G). Finally, an ELISpot assay performed using a 15-mer peptide pool that tiled the sequence of the BA.5 spike protein confirmed that, in the group administered the mRNA vaccine prepared using ψUTP and UTP, the spot forming unit (SFU) value, which indicates the number of activated T cells secreting IFN-γ, was high at approximately 2000, compared to the negative control (FIG. 9H).

### 10. Evaluation of the viral infection defense efficacy of the double-mutated spike antigen with inhibited cleavage

To evaluate the efficacy of the Omicron BA.5-based mRNA-LNP vaccine described above, the vaccine was administered intramuscularly into the hind limb of BALB/c mice (6 mice in the placebo group and 5 in the vaccine group) at two-week intervals for two doses. High-titer RBD-binding antibodies (~10⁶ endpoint titer) were confirmed via ELISA using serum collected two weeks after the booster dose (FIG. 10B). Nineteen days after the booster vaccination, mice were challenged intranasally with 1×10⁵ PFU of Omicron BA.5 (B.1.1.529.5) variant viruses (FIG. 10A).

Furthermore, two days post-challenge, viral RNA and infectious viruses were detected in lung tissue from the placebo group but were below detection limits in the vaccine group (FIG. 10C). Immunohistochemistry (IHC) analysis revealed no nucleocapsid protein (N) expression in the lung tissues of the vaccine group (FIG. 10D, upper panel). Additionally, hematoxylin and eosin (H&E) staining demonstrated significantly reduced lung lesions in the vaccinated mice (FIG. 10D, lower panel), and the levels of the inflammatory cytokine IL-6 were notably lower in this vaccinated group as well (FIG. 10D, right bar graph).

### 11. Evaluation of cross-immune responses of the BA.5 variant spike-targeting vaccine with double mutations to other variants

Neutralization assays using serum from vaccinated mice (Example 9) and pseudotyped viruses expressing spike proteins from other variants revealed that the neutralization capacity for BQ.1, BA.2.75.2, and XBB.1 variants were reduced compared to BA.5. However, the ND50 value for XBB.1, which was known for its strong ability to evade antibodies generated against BS.5 spike proteins, was found to be 521, suggesting that the vaccine may still confer a considerable level of protection against this variant over an extended period (FIG. 11). It was also confirmed that there was no significant difference in cross-neutralization against multiple variants between the mRNA vaccines produced using ψUTP and UTP, as shown by the comparison of relative neutralization ratios for each variant (the comparison of relative neutralization ratio for each variant is displayed at the bottom of the table in FIG. 11)

### 12. Evaluation of the immunogenicity of the XBB.1.5 variant modified spike antigen with double mutations

To assess the immunogenicity of the modified spike proteins, which included substitutions of R685S and R815M (hereinafter, SM substitution), which differ from the double mutations described in Examples 9, 10, and 11, mRNA expressing the modified spike protein with the corresponding double mutations was synthesized using unmodified UTP and formulated into LNPs, in the same manner as described in Example 9. The formulated vaccine was administered intramuscularly to mice at a dose of 1 µg, with two injections given three weeks apart (FIG. 12A).

Subsequently, two weeks after the second vaccination, serum antibody titers were analyzed using ELISA. The XBB.1.5 spike protein with the SM substitution (XBB.1.5_SM) elicited high levels of antibodies, with titers against the full-length spike protein and the receptor-binding domain (RBD) exceeding 10⁷ (FIG. 12B). In addition, neutralizing antibody titration using pseudotyped viruses revealed that the ND₅₀ value, representing the serum dilution required for 50% virus neutralization efficiency, exceeded 10,000 in the vaccine-injected group, outperforming the negative control (FIG. 12C).

## Claims

1. A spike antigen protein of coronavirus, in which a host protease cleavage site of the spike protein of coronavirus is modified.

2. The spike antigen protein of claim 1, wherein the cleavage site is at least one selected from the cleavage sites of the furin protease in the sequences of the S1 and S2 domains of the spike protein of coronavirus and the cleavage site of the protease in the S2' sequence thereof.

3. The spike antigen protein of claim 1, wherein the coronavirus is at least one selected from the group consisting of severe acute respiratory syndrome coronavirus1 (SARS-CoV), severe acute respiratory syndrome coronavirus2 (SARS-CoV-2), Middle East respiratory syndrome coronavirus (MERS-CoV), human coronavirus 229E (HCoV-229E), and variants thereof.

4. The spike antigen protein of claim 2, wherein the coronavirus is SARS-CoV-2, the furin protease cleavage site is at amino acid 685 of the spike protein, and the protease cleavage site in the S2' sequence is at amino acid 815 of the spike protein.

5. The spike antigen protein of claim 4, wherein amino acid 685 at the furin protease cleavage site is substituted with at least one amino acid selected from the group consisting of glycine, alanine, valine, leucine, isoleucine, threonine, serine, cysteine, methionine, aspartic acid, asparagine, glutamic acid, glutamine, lysine, histidine, phenylalanine, tyrosine, tryptophan, and proline.

6. The spike antigen protein of claim 4, wherein, at the cleavage site of the protease in the S2' sequence, amino acid 815 of the spike protein is substituted with at least one amino acid selected from the group consisting of glycine, alanine, valine, leucine, isoleucine, threonine, serine, cysteine, methionine, aspartic acid, asparagine, glutamic acid, glutamine, phenylalanine, tyrosine, tryptophan, and proline.

7. The spike antigen protein of claim 1, wherein the modified spike antigen protein of coronavirus inhibits the generation of a free S1 domain.

8. The spike antigen protein of claim 1, wherein the modified spike antigen protein of coronavirus inhibits cell membrane fusion.

9. The spike antigen protein of claim 1, wherein the modified spike antigen protein of coronavirus inhibits intracellular entry of the virus or intracellular transfer of the viral genome.

10. A recombinant vector for expressing an antigen protein of coronavirus, comprising a gene encoding the spike antigen protein of coronavirus of any one of claims 1 to 9.

11. A transformant which is transformed with the recombinant vector of claim 10.

12. A vaccine composition against coronavirus, comprising the spike antigen protein of coronavirus of any one of claims 1 to 9, or a gene encoding the same.

13. The vaccine composition of claim 12, wherein the vaccine composition increases the ability to produce a neutralizing antibody.

14. A pharmaceutical composition for preventing or treating coronavirus infection, comprising the spike antigen protein of coronavirus of any one of claims 1 to 9.

15. A composition for diagnosing coronavirus infection, comprising the spike antigen protein of coronavirus of any one of claims 1 to 9.

16. A coronavirus infection diagnostic kit, comprising the composition of claim 15.

17. A method of introducing an antigen gene of coronavirus, comprising transforming a gene which encodes the spike antigen protein of coronavirus of any one of claims 1 to 9 into a subject.

18. A method of preventing or treating coronavirus infection, comprising administering the spike antigen protein of coronavirus of any one of claims 1 to 9 to an individual in need thereof.
